# EUROPEAN PATENT APPLICATION

(11) **EP 1 167 532 A2**
(43) Date of publication of application: **02.01.2002**
(21) Application number: 01305574.4
(22) Date of filing: 27.06.2001
(51) Int. Cl.: C12N 15/82

(54) **Edible transgenic plants expressing mammalian proteins**

(30) Priority: 30.06.2000 JP 2000200195
(71) Applicant: KABUSHIKI KAISHA HAYASHIBARA SEIBUTSU KAGAKU KENKYUJO, Okayama-shi Okayama (JP); Sugimoto, Chihiro, Sapporo-shi, Hokkaido 063-0062 (JP); Hokkaido Green-Bio Institute, Inc., Hokkaido 069-1317 (JP); THE HOKUREN FEDERATION OF AGRICULTURAL COOPERATIVES, Sapporo, Hokkaido (JP)
(72) Inventor: Ikegami, Hakuo, Okayama 719-1135 (JP); Kurimoto, Masashi, Okayama-shi, Okayama 700-0013 (JP); Sugimoto, Chihiro, Sapporo-shi, Hokkaido 063-0062 (JP); Matsumura, Takeshi, Sapporo-shi, Hokkaido 004-0022 (JP)
(74) Representative: Daniels, Jeffrey Nicholas

(57) **Abstract**

Disclosed are transgenic plants produced from edible plants by transforming them with a DNA encoding one or more physiologically active proteins, including cytokines, of mammalian origin. The transgenic plants contain the physiologically active proteins of mammalian origin, and the proteins present in the transgenic plants exhibit efficacy for preventing intractable diseases in the bodies of mammals that ingested the plants without causing serious side effects. The transgenic plants are useful as healthcare foods that are daily feasible for the aim of preventing intractable diseases such as malignant tumors.

## Description

This invention relates to novel transgenic plants, more particularly, transgenic plants produced from edible plants by transforming them with a DNA encoding one or more physiologically active proteins of mammalian origin.

The modern medicine is in remarkable progress. Various medical treatments have been developed and proved efficacious against many diseases which had been deemed intractable; some treatments have succeeded in eradicating their target diseases. However, considerable numbers of diseases remain intractable, as seen in malignant tumors. Patients suffering from such diseases are usually treated with various therapies dependently on their symptoms. Such therapies often restrict the patients' daily lives stringently.

Under these circumstances, people desire to prevent intractable diseases surely and easily before suffering from such diseases. However, most of popular ways for preventing diseases are mere a regimen for health, such as taking a proper amount of exercise, balanced diet, etc., which do not necessarily result in prevention of intractable diseases.

Therapies with cytokine and other physiologically active proteins exhibit distinguished efficacy against certain diseases, but such proteins are neither easily obtainable nor usable for ordinary people. In addition, such proteins may exhibit not only desirable efficacy, but also unacceptable side effects in some patients. The established therapies with cytokine and other physiologically active proteins, therefore, would not be responsive to the desire for preventing intractable diseases surely and easily.

In view of the forgoing, an aim of this invention is to provide products easily usable in daily life that exhibit efficacy for preventing intractable diseases including malignant tumors.

The present inventors focused on the actions of physiologically active proteins, and assumed that the above object can be attained by developing edible products such as healthcare foods containing physiologically active proteins that moderately exhibit their activities in mammalian bodies when ingested throgh oral routes. However, the physiologically active proteins are not easily usable for ordinary people. The present inventors conceived from these circumstances to produce edible plants containing physiologically active proteins of mammalian origin by means of genetic engineering techniques.

The present inventors transformed cells of edible plants with DNAs encoding physiologically active proteins of mammalian origin, and regenerated plant bodies from the transformants, resulting in establishment of transgenic plants. The transgenic plants expressed the physiologically active proteins in active forms in their plant bodies. The transgenic plants were examined for the effects in experimental animals by orally administering the plants. The proteins exhibited their inherent activities also in the animal bodies, and physiological functions of the animals were expectedly modulated, while serious side effect was not observed. Further, the efficaciousness of the transgenic plants in oral administration were studied in comparison with isolated preparations of the physiologically active proteins, purified at a level feasible for pharmaceutical uses. As a result, the efficaciousness of the transgenic plants was proved higher than the cases of the isolated preparations and compositions comprising such a preparation. From these facts, the present inventors concluded that when animals ingest the transgenic plants containing physiologically active proteins of mammalian origin, the proteins exhibit desirable efficacy in the animal bodies. This invention is established based on these findings.

As described above, this invention attains the above object by providing transgenic plants produced from edible plants by transforming them with a DNA encoding one or more physiologically active proteins of mammalian origin.

### Detailed Description of the Invention

This invention provides transgenic plants produced from edible plants by transforming them with a DNA encoding one or more physiologically active proteins of mammalian origin. The physiologically active proteins of mammalian origin as referred to in this invention include, regardless of their molecular types or origins, those inherently produced in mammalian bodies as a substance responsible for the maintenance or modulation of physiological functions in mammals, and also exhibiting the inherent activity when given in transgenic plant form to humans or other animals through oral routes. Examples of such physiologically active proteins are cytokines, more particularly, interferons, interleukins, hematopoietic factors, and growth factors of mammalian origin. Among these proteins, interferons including interferon α and hematopoietic factors including erythropoietin are particularly useful, because their efficaciousness in mammalian bodies when ingested as an transgenic plants is relatively higher level than others without causing serious side effects.

This invention should not be limited to transgenic plants with physiologically active proteins of specific origins, as mentioned above; it is preferable to employ proteins originated from an mammal expected to ingest the transgenic plants of this invention, or from other animals genetically related thereto, because some proteins express their activities in a species-specific manner. Preferable examples of the origin include human and other primates, bovine, porcine, and other artiodactyls, and mouse, rat, and other rodents.

To practice this invention, a DNA encoding the physiologically active protein(s) of mammalian origin is first prepared. Such a DNA can be prepared from appropriate sources according to usual DNA cloning methods such as hybridization, immunological screening, PCR, etc. In such cloning, conventional databases of nucleic acids such as "GenBank®," administrated and managed by National Institute of Health (NIH), USA, are useful because they contain information useful for cloning on various DNAs, for example, nucleotide sequences, sources of DNAs.

While the DNAs prepared as above can be directly used by inserting them into vectors for transforming plants (described below), the DNAs can be used, if necessary, after altering them by addition, replacement, and/or deletion of nucleotide(s) in the coding sequence thereof and/or neighboring sequences at the 5'-upstream and/or 3'-downstream of the coding sequences.

Regulatory sequences for DNA expression such as promoters and enhancers can be added to the coding sequences. The regulatory sequences can be classified as follows: constitutive regulatory sequences, promoting DNA expression under any circumstances; inducible regulatory sequences, inducing DNA expression dependently on external conditions or differentiation stages of cells; and tissue-specific regulatory sequences, promoting or enhancing DNA expression in a tissue-specific manner. An example of the constitutive regulatory sequence is 35S promoter of cauliflower mosaic virus; the inducible regulatory sequence, the photo-specific regulatory sequence of ribulose-bisphosphate carboxylase small subunit gene; and the tissue-specific regulatory sequence, the seed-specific regulatory sequence of phaseolin gene. From these regulatory sequences, suitable one(s) can be selected depending on the race of host plant and the uses of the resulting transgenic plants. For example, the growth of the transgenic plants in some races may be affected by constitutive expression of the transferred DNA all over the plant body; such a problem can be solved by an inducible or tissue-specific regulatory sequence attached to the coding sequences.

The DNAs encoding the physiologically active proteins of mammalian origin can be altered in the coding sequences not to change the amino acid sequences inherently encoded thereby. Some plants have characteristics different from others in their genomes, for example, specific GC contents, codon usages, etc. When a host plant has such characteristics, altering the DNAs in the coding sequences before use in accordance with the characteristics may stabilize the expression of the DNAs in the transgenic plants. In addition, inserting a plant gene intron(s) into the coding sequences of the DNAs may enhance or stabilize the DNA expression. Therefore, the insertion of such introns can be also beneficially conducted in this invention.

To the DNAs encoding the physiologically active proteins of mammalian origin, a coding sequence for a signal peptide or sorting signal into organelle can be added. Most of the physiologically active proteins of mammalian origin are secretory proteins, which usually contain a signal peptide at the N-termini. While the inherent signal peptide can be used in this invention, replacing inherent sequence with a signal peptide from other origin such as yeast α-factor signal peptide may increase the secretion level of the physiologically active proteins of mammalian origin in plant bodies. Therefore, a DNA for a signal peptide selected dependently on the uses of the transgenic plants can be added to the coding sequences. In addition, a sorting signal into organelle, added to the coding sequence, may stabilize the intracellular expression of the DNAs without affecting the activities of the expressed proteins. Examples of the sorting signals useful in this invention include endoplasmic reticulum retention signal, chloroplast sorting signal, and mitochondrion sorting signal.

A variety of procedures for cloning DNAs and altering cloned DNAs are described, for example, in J. Sambrook et al., "Molecular Cloning, A Laboratory Manual, Second edition," (published by Cold Spring Harbor Laboratories, 1989). To prepare the DNAs to be used in this invention, such conventional procedures can be arbitrarily employed.

The transgenic plants of this invention can be produced by plant transformation with the DNAs preparable as above. The transformation can be accomplished by means of, roughly classified, biological technique or direct DNA transfer technique. Examples of the biological technique include DNA transfer methods by mediation of a microorganism of the genus *Agrobacterium* (hereinafter called "*Agrobacterium*"). Examples of the direct DNA transfer technique include methods to directly transfer a DNA into plant protoplasts, cells, or tissues. From these methods, a suitable one for the race of the host plant to be transformed can be selected. The following show the outline of the procedures for practicing this invention by each method.

To transfer the DNA prepared for this invention (hereinafter, may be called "the foreign DNA") into plants by mediation of *Agrobacterium*, *Agrobacterium* is first manipulated into the form containing Ti plasmid in which T-DNA region is replaced by the foreign DNA. *Agrobacterium* in such a form can be obtained by either so-called "intermediate vector method" or "binary vector method." In the former method, an intermediate vector comprising the foreign DNA is first prepared in a usual manner, and then the intermediate vector is introduced into *Agrobacterium* carrying Ti plasmid which has been preliminarily engineered to comprise non-pathogenic T-DNA, so that the foreign DNA can be inserted into the T-DNA region by homologous recombination. In the latter method, a binary vector designed to comprise the replication origin effecting in *Agrobacterium,* if necessary, together with that effecting in *Escherichia coli,* and the border sequences of T-DNA between which the foreign DNA intervenes is first prepared in a usual manner, and then the binary vector is introduced into *Agrobacterium* carrying Ti plasmid lacking T-DNA. Intermediate vectors and binary vectors are preferably designed to contain selection markers, for example, neomycin phosphotransferase gene, involved in kanamycin resistance, chloramphenicol acetyltransferase gene, involved in chloramphenicol resistance, etc. The vectors Bin19 (ATCC 37327), pAK1003 (ATCC 37425), and pAP2034 (ATCC 37428) are preferable examples for either method. Other intermediate vectors and binary vectors are described in "Methods in Enzymology," Vol. 153 (edited by Ray Wu et al., published by Academic Press Inc., 1987), chapter 15. Vectors prepared similarly as in this publication can be also used arbitrarily in this invention.

By infecting plants with *Agrobacterium* prepared as above, containing the foreign DNA, the foreign DNA can be transferred into the plants. Infecting plants with *Agrobacterium* can be accomplished by incubating with this microorganism an appropriate type of host selected depending on the race of the plant to be transformed, for example, plant tissues, calli, cells, and protoplasts. From the hosts that received the infection, plant bodies can be regenerated by incubating the hosts with an appropriate inducer for plant differentiation such as auxin. After these procedures, the regenerated plants can be examined on their chromosomal DNAs by PCR, Southern blotting, etc., and on the expression of the foreign DNAs. The establishment of the transgenic plants can be judged by the results showing the integration of the foreign DNA into the chromosomal DNAs and the expression of the foreign DNA under desired conditions.

These methods for transforming plants by mediation of *Agrobacterium* are useful for producing the transgenic plants from *Agrobacterium*-sensitive plants, for example, most of dicots including tomatoes and lettuces and some of monocots including rices and corns. Procedures for the above methods are described in detail, for example, in "Methods in Enzymology," Vol. 153 (edited by Ray Wu et al., published by Academic Press Inc., 1987), chapters 16 to 18. Such conventional procedures can be arbitrarily employed in this invention.

Transforming plants by direct transfer of the foreign DNA can be accomplished, for example, by the method using particle gun (biolistic method), electroporation method, and polyethylene glycol method. In biolistic method, the foreign DNA is first made into a recombinant DNA by inserting the foreign DNA into an appropriate vector, usually, a type of plasmid vector replicable in *Escherichia coli*, if necessary, comprising selection markers. Tissues, cell cultures, calli, or protoplasts are prepared from the objective plants in a usual manner for the use as the hosts for DNA transfer. Subsequently, after heavy metal particles are coated with the recombinant DNA comprising the foreign DNA, the particles are bombarded to the hosts by using a conventional particle gun (for example, commercialized by DuPont®), effecting with an appropriate propellant such as helium gas, under conditions according to the manufacturer's instructions, usually, to give a velocity of several hundred meters per second. Examples of the heavy metal particles include gold and tungsten particles. From the bombarded hosts, plant bodies can be regenerated by cultivating the hosts under appropriate conditions depending on the race of the hosts and further cultivating in the presence of an inducer for plant differentiation such as auxin. Similarly as in the method by mediation of *Agrobacterium,* the establishment of the transgenic plants can be judged by experimental results showing the integration of the foreign DNA and the expression of the foreign DNA in the regenerated plant bodies. Biolistic method has an advantage that this method can be applied to any race of plant.

In electroporation method, the foreign DNA is first made into a recombinant DNA similarly as in biolistic method. It is preferable to linearize the recombinant DNA before use, for example, by means of restriction enzymes. Protoplasts are prepared from the objective plants in a usual manner for the use as the hosts for DNA transfer. Subsequently, the protoplasts are suspended in an appropriate salt solution made isotonic or hypertonic with appropriate solutes such as mannitol, heated at about 45° C, and then mixed with the recombinant DNA comprising the foreign DNA, along with carrier DNAs such as bovine thymus DNA, and the mixture are loaded with an electric pressure using a conventional electroporator (for example, commercialized by DIA-LOG GmbH., Germany). It is preferable to preliminarily optimize the electric pressures by experiments with reference to usual conditions, because some races of plants prefer to an electric pressure for receiving DNA transfer different from usual conditions. From the protoplasts loaded with the electric pressures, plant bodies can be regenerated by cultivating the protoplasts on soft agar media and subsequently cultivating the generated colonies in the presence of an inducer for plant differentiation such as auxin. In polyethylene glycol method, the foreign DNA can be prepared similarly as in electroporation method, and the foreign DNA transfer can be accomplished by the steps as follows: suspending protoplasts as the hosts in appropriate media, heating the suspension similarly as in electroporation method, mixing the suspension with the foreign DNA and carrier DNAs and then with polyethylene glycol dissolved in appropriate liquid media, and gently stirring the resulting mixture for about 30 minutes. Thereafter, plant bodies can be regenerated by cultivating the protoplasts similarly as in electroporation method. Similarly as in the method by mediation of *Agrobacterium,* the establishment of the transgenic plants can be judged by experimental results showing the integration of the foreign DNA and the expression of the foreign DNA in the regenerated plant bodies.

Both methods using electroporation and polyethylene glycol are useful for producing the transgenic plants of this invention from plants whose protoplasts can be prepared by established methods, for example, corns, wheats, rices, etc. Procedures for these methods are described in detail, for example, in "Methods in Enzymology," Vol. 153 (edited by Ray Wu et al., published by Academic Press Inc., 1987), chapters 19 to 21. Such conventional procedures can be arbitrarily employed in this invention.

The transgenic plants of this invention obtainable as above contain the physiologically active proteins of mammalian origin in their plant bodies, preferably, in tissues usually feasible as foods. The contents of the physiologically active proteins in the transgenic plants are not limited to specific ranges as far as they exhibit the desirable efficacy in mammalian bodies when the plants are ingested by mammals. The contents of the proteins in the transgenic plants are usually 0.1 ng or higher, preferably, 0.1 µg or higher, per one kilogram by fresh weight, while the values may vary depending on the race of the transgenic plants or methods of transformation. The upper bounds of the contents, which are not limited in view of surely exerting the activities of the physiologically active proteins, are usually one milligram or lower, considering the races of the transgenic plants and methods of transformation feasible in this invention.

The transgenic plants of this invention can be produced from desired plants, as mentioned above, by methods appropriately selected depending on the race of the plants to be transformed. While the races of the transgenic plants are not limited to specific ones as far as they are usable as foods for humans or other animals, in view of surely exerting the activities by orally ingesting the plants, it is relatively preferable to use the races edible without heating or cooking for a mammal that is expected to ingest the transgenic plants of this invention. For applying to humans, examples of the preferable plant races include those of the families Asteraceae, Brassicaceae, Cucurbitaceae, Apiaceae, Rosaceae, Vitaceae, Ericaceae, Caricaceae, Fabaceae, Juglandaceae, Chenopodiaceae, and Solanaceae, more specifically, lettuces, chicories, *Artemisia* sp., broccolis, cabbages, radishes, horseradishes, mustards, cucumbers, melons, pumpkins, chayotes, carrots, Japanese hornworts, celeries, apples, plums, Japanese apricots, peaches, strawberries, raspberries, almonds, pears, loquats, grapes, cranberries, mountain cranberries, blueberries, papayas, alfalfas, soybeans, walnuts, spinaches, tomatoes, and redpeppers. For applying to animals other than humans, examples of the preferable plant races include, in addition to the above-exemplified plants, those of the families Convolvulaceae, Poaceae, and Discoreaceae, more specifically, sweet potatoes, rices, corns, wheats, barleys, ryes, Japanese yams, and potatoes.

The transgenic plants of this invention can be provided in the intact form as they are harvested or the form of isolated tissues of leaves, stems, roots, fruits, peels, buds, seeds, petals, or other edible tissues. If necessary, the transgenic plants can be provided after further processed by cutting, peeling, pulverizing, squeezing, extracting, or any other step into the form of cut vegetables, cut fruits, powders, juice, extracts, etc. The transgenic plants, in any form, can be treated with appropriate additives to maintain the freshness of plants, before providing. Examples of such additives include saccharides, vitamins, and antioxidants. Particular examples of saccharides feasible for this use are as follows: monosaccharides such as glucose, sorbitol, mannose, fructose, galactose, and galactitol; oligosaccharides such as maltose, maltitol, sucrose, trehalose, lactose, maltotriose, and maltotetraose; and polysaccharides such as starch, starch partial hydrolyzates, pullullan, amylopectin, amylose, pectin, and mannan. Among these saccharides, those having lower molecular weights are relatively preferable, and non-reducing, lower molecular weight saccharides, for example, trehalose and maltitol, are more preferable, because the effectiveness of them are relatively higher than others. In addition, the methods disclosed in Japanese Patent Kokai Nos.224,565/97 and 252,719/97, for maintaining the freshness of cut vegetables by contacting a composition of trehalose with such vegetables, are useful in providing the transgenic plants in the form of cut vegetables or cut fruits. Commercially available trehalose products in food grade (for example, "Treha™," commercialized by Hayashibara Shoji Co., Ltd., Okayama, Japan) are useful for these methods. In addition, because trehalose is expected to improve the stability of proteins in general, the above methods are beneficially conducted for maintaining the activities of the physiologically active proteins of mammalian origin contained by the transgenic plants of this invention.

The transgenic plants of this invention as described above are applicable to humans as well as to domestic animals, poultries, pets, and other animals. While the ways of use are not limited to specific ones, it is preferable to use the transgenic plants without cooking or heating for exerting the activities of the physiologically active proteins of mammalian origin in the transgenic plants as effectively as possible.

The transgenic plants are more advantageous than the physiologically active proteins for pharmaceutical uses such as injections, because the desired effects exhibited by the transgenic plants are moderate without serious side effects. In addition, the transgenic plants have an advantage that in the use of oral ingestion, these plants can exhibit the desirable efficacy more surely than the physiologically active proteins for pharmaceutical uses in the form of itself alone or pharmaceutical compositions. The transgenic plants of this invention are, therefore, particularly useful as health foods for humans and other mammals to maintain or enhance their health.

The following Examples explain this invention in further detail. Because these Examples can be modified by the technical level of this field, the scope of this invention should not be limited to these Examples.

### Example 1

### Transgenic tomato

Transgenic tomato with a human interferon α gene was produced by the DNA transfer method by mediation of *Agrobacterium* as follows.

### Example 1-1

### Preparation of Agrobacterium for foreign DNA transfer

A recipient strain of *Agrobacterium* for the transfer of a foreign DNA into plants was prepared by manipulating *Agrobacterium tumefaciens* according to the methods described in R. Deblaere et al., "Nucleic Acid Research," Vol. 13, P.4777 (1985). This recipient strain has Ti plasmid that lacks the border sequences and other sequences in T-DNA region.

A binary vector to transfer a foreign DNA was prepared according to the methods for preparing the binary vector PGSJ280 described in "Methods in Enzymology," Vol. 153 (edited by Ray Wu et al., published by Academic Press Inc., 1987), chapter 16. This vector comprises the border sequences of T-DNA with the intervention by cauliflower mosaic virus 35S promoter, polyadenylation signal derived from T-DNA, and neomycin phosphotransferase gene for kanamycin resistance.

From the nucleic acid database "GenBank®," the information on the human interferon α2 gene registered under accession No.Y11834 was obtained. A usual PCR was carried out with primers designed with reference to this information and a human genomic DNA preparation as a template to amplify the coding sequence for human interferon α along with its signal peptide. The amplified DNA fragment was inserted into the above-prepared binary vector at the downstream of cauliflower mosaic virus 35S promoter. The resulting recombinant DNA was introduced into the recipient strain of *Agrobacterium,* prepared above, in a usual manner. Thus, *Agrobacterium* was prepared for transferring the human interferon α gene as a foreign DNA.

### Example 1-2

### Plant transformation

According to the methods described in "Methods in Enzymology," vol. 153 (edited by Ray Wu et al., published by Academic Press Inc., 1987), chapter 16 for transforming tobacco leaves, tomato leaves were transformed by mediation of *Agrobacterium,* prepared above, for the foreign DNA transfer. Tomato leaves were cut into about 0.5 cm² discs, and floated on liquid MS medium. To the medium, 1/100 volume of the culture of *Agrobacterium* prepared in Example 1-1 was added, and then the medium was incubated at 25°C for two days in a plant cultivator to infect the leaves with *Agrobacterium*. Thereafter the leaves were washed with a fresh preparation of liquid MS medium, transferred onto MS agar medium containing cefotaxime and kanamycin as antibiotics and auxin as an inducer for plant differentiation, prepared in a usual manner, and incubated to induce roots from the leaves at 25°C for three weeks in a plant cultivator under the lightening cycle of 16-hour-lightness and 8-hour-darkness. The tomato leaves with roots induced were transferred onto auxin-free MS agar medium and incubated similarly as above to regenerate juvenile plant bodies. From each juvenile plant body, a part was collected and homogenized, and the homogenates were examined for the existence of the human interferon α gene by a usual PCR. Plant bodies which were observed to integrate the gene were selected as those of transgenic tomato.

The above-selected juvenile plant bodies of the transgenic tomato were planted in a test garden. The tomato fruits formed were harvested, and the harvested product was homogenized and extracted with the equal volume of an appropriate extraction buffer (pH 8.0) prepared in a usual manner. The resulting extract was centrifuged, and the supernatant was concentrated by ultrafiltration. The concentrate was partially purified by usual methods for interferon α purification. The partially purified preparation was examined for anti-viral activity by the assay using human FL cells and Sindbis virus. The supernatant of the extract was estimated to contain about 35 international units of anti-viral activity per one milliliter.

An animal experiment was conducted using two groups of C3H/HeN mice (10 heads per group, age of six-week-old, body weight from about 20 g to about 30 g) as follows. To the mice of group 1 (test group), the fruits of the transgenic tomato, planted and harvested as above and cut into pieces, were fed at one gram by wet weight per head. To the mice of group 2 (control group), tomato fruits with no foreign DNA were fed similarly as in group 1. On day 14 from the start of this experiment, all mice were weighed, and then from each mouse, the blood was gathered. Each blood was examined for natural killer activity by a usual assay using ⁵¹Cr-labeled L₉₂₉ cells as target cells. The test group mice showed the activity at a level remarkably higher than those of the control group mice did, while the body weights of the two groups were not significantly different.

Another experiment was carried out by the procedure as above except for using as experimental animals C3H/HeN mice infected with vaccinia virus at 200 pfu/head. On day 14 from the start of this experiment, all mice were examined for the numbers of eruption at the tails. The test group mice showed the eruption counts at a level apparently lower than those of the control group mice.

The results of the above animal experiments indicate that human interferon α contained in the transgenic tomato of this Example enhanced the immunological potency of mice that ingested the plants, without causing serious side effects. From these results, the transgenic tomato of this Example is expected to enhance the immunological potency of humans and other mammals when used as a food, and is therefore useful as a health food to prevent serious diseases including malignant tumors.

### Example 2

### Transgenic carrot

Transgenic carrot with a human interferon α gene was produced by DNA transfer method by mediation of *Agrobacterium* as follows.

According to Example 1-1, *Agrobacterium* was manipulated for transferring the human interferon α2 gene as a foreign DNA. A callus culture of carrot in logarithmic growth phase was prepared in a usual manner, and transferred into sterilized Petri dishes. To the dishes, an appropriate amount of the culture of above-prepared *Agrobacterium* was added, and the dishes were incubated at 28°C for two days. Thereafter the carrot calli were washed with a fresh preparation of an appropriate liquid medium, and then regenerated into juvenile plant bodies in a usual manner, by incubating in a medium containing kanamycin and cefotaxime as antibiotics and auxin as an inducer for plant differentiation for three weeks. From each juvenile plant body, a part was collected and homogenized, and the homogenates were examined for the existence of the human interferon α gene by a usual PCR. Plant bodies which were observed to integrate the gene were selected as those of the transgenic carrot.

The above-selected juvenile plant bodies of the transgenic carrot were planted in a test garden. The edible parts (underground tissues) were harvested from the carrots that had grown up, and the harvested product was homogenized and extracted with the equal volume of an appropriate extraction buffer (pH 8.0). The extract was centrifuged, and the supernatant was concentrated by ultrafiltration. The concentrate was partially purified by usual methods for interferon α purification. The partially purified preparation was examined for anti-viral activity by the assay using human FL cells and Sindbis virus. The supernatant of the extract was estimated to contain about 27 international units of anti-viral activity per one milliliter.

An animal experiment was conducted with two groups of C57BL/6 mice (10 heads per group, age of six-week-old, body weight from about 20 g to about 30 g). To the mice of group 1 (test group), the transgenic carrot, planted and harvested as above and cut into pieces, was fed at one gram by fresh weigh per head per day. To the mice of group 2 (control group), a carrot with no foreign DNA was fed similarly as in group 1. On day 14 from the start of this experiment, all mice were weighed, and then from each mouse, the blood was gathered. Each gathered blood was examined for natural killer activity by a usual assay using YAC-1 cells as target cells. The obtained values were averaged in each group. The mean of the test group was about 1.7-fold of that of the control group, while the body weights of the two groups were not significantly different. These experiments indicate that human interferon α contained in the transgenic carrot of this Example enhanced the immunological potency of the mice without causing serious side effects. Another experiment was conducted similarly as the above experiment in which a usual feed containing an isolated preparation of human interferon α was used in place of the transgenic carrot to feed mice at a daily dose corresponding to the above test group with respect to the amount of interferon α. In this experiment, the effect was not apparent as in the above test group. This result indicates that in the uses through oral routes, the transgenic plants of this invention exhibit the desired effects more surely than isolated preparations of the physiologically active proteins.

From these results, the transgenic carrot of this Example is expected to enhance the immunological potency of humans and other mammals when used as a food, and is therefore useful as a health food to prevent intractable diseases including malignant tumors.

### Example 3

### Transgenic lettuce

Transgenic lettuce with a human interferon α gene was produced by electroporation method as follows.

### Example 3-1

### Preparation of recombinant DNA for foreign DNA transfer

According to Example 1-1, a DNA fragment consisting of the coding sequence for the human interferon α2 gene was amplified by PCR. This DNA fragment was inserted into the plasmid vector pUC8 in a usual manner. The resulting recombinant DNA was further manipulated in a usual manner to comprise the photo-specific regulatory sequence of pea ribulose-bisphosphate carboxylase small subunit at the upstream of the inserted DNA fragment and chloramphenicol acetyltransferase gene at a different site. The obtained recombinant DNA was linearized by restriction enzyme treatment, and thereafter purified by cesium chloride density-gradient centrifugation for the use of transferring the foreign DNA.

### Example 3-2

### Plant transformation

Lettuce leaves were treated with cellulase in a usual manner to prepare lettuce protoplasts. The transfer of the foreign DNA into the lettuce protoplasts and the regeneration of plant bodies from the protoplasts were conducted according to transformation of tobacco protoplasts described in "Methods in Enzymology," Vol. 153 (edited by Ray Wu et al., published by Academic Press Inc., 1987), chapter 19. First, lettuce protoplasts were suspended in a buffer (pH 5.6) containing mannitol (0.4 M), magnesium chloride (6 mM), and 2-morpholino ethanesulphonic acid (MES) (1 g/l) to give a cell density of about 2 × 10⁶ cells/ml. The suspension was incubated at 45°C for five minutes and then cooled on ice to room temperature. After 0.25 ml of the suspension was transferred into a polycarbonate tube, and to the tube, four micrograms of the recombinant DNA prepared in Example 3-1 was added together with 20 µg of calf thymus DNA as a carrier DNA and 0.13 ml of 24%(w/v) polyethylene glycol solution. The resulting mixture was loaded with an electric pressure at 1.5 kV/cm in a usual manner to transfer the foreign DNA into the protoplasts.

The protoplasts with the foreign DNA transferred were cultivated to form colonies according to agarose bead type culture system described in R. D. Shillito et al., "Plant Cell Reports," Vol. 2, p. 244 (1983) with a medium formulated in a usual manner to contain chloramphenicol. The colonies formed were independently collected and transferred into LS medium containing auxin for inducing roots from the colonies, and subsequently transferred into T medium to regenerate juvenile plant bodies. From each juvenile plant body, a part was collected and homogenized, and the homogenates were examined for the existence of the human interferon α gene by a usual PCR. Plant bodies which were observed to integrate the gene were selected as those of the transgenic lettuce.

The above-selected juvenile bodies of the transgenic lettuce were planted in a test garden. The edible parts (aboveground parts) were harvested from the plants that had grown up, and the harvested product was homogenized and extracted with the equal volume of an appropriate extraction buffer (pH 8.0) prepared in a usual manner. The extract was centrifuged, and the supernatant was concentrated by ultrafiltration. The concentrate was partially purified according to usual methods for interferon α purification. The partially purified preparation was examined for anti-viral activity by the assay using human FL cells and Sindbis virus. The supernatant of the extract was estimated to contain about 17 international units of anti-viral activity per one milliliter.

An animal experiment was conducted with two groups of NOD mice (10 heads per group, age of six-week-old, body weight from about 20 g to about 30 g) as follows. To the mice of group 1 (test group), the leaves of the transgenic lettuce, planted and harvested as above and cut into pieces, were fed at one gram by fresh weight per head per time with a frequency of three times per week. To the mice of group 2 (control group), lettuce leaves with no foreign DNA were fed similarly as in group 1. According to this feeding schedule, each group mice were housed for 38 weeks. Thereafter the blood was gathered from each mouse. In a usual manner, each group was analyzed for the mean value of the blood sugars and the ratio of diabetes crisis. The blood sugar value and the ratio of diabetes crisis of the test group were remarkably lower than those of the control group. These results indicate that human interferon α contained in the transgenic lettuce of this Example enhanced the immunological potency of the mice, and modulated their physiological functions.

From these results, the transgenic lettuce of this Example is expected to enhance the immunological potency of humans and other mammals when used as a food, and is therefore useful as a health food to prevent intractable diseases including malignant tumors.

### Example 4

### Transgenic strawberry

Transgenic strawberry with a human erythropoietin cDNA was produced by the DNA transfer method by mediation of *Agrobacterium*.

### Example 4-1

### Preparation of Agrobacterium for foreign DNA transfer

From the nucleic acid database "GenBank®," the information on the nucleotide sequence of the cDNA for human erythropoietin registered the accession No.X02157 was obtained. A usual PCR was carried out with primers designed with reference to this information and a commercially available human fetal liver poly (A)⁺ RNA as a template to amplify the cDNA coding for mature erythropoietin of human origin. A DNA coding for the signal peptide of yeast α-factor was amplified by a usual PCR. The amplified cDNA and DNA were inserted into the binary vector used in Example 1-1 so that the cDNA was located at the downstream of the DNA coding for the signal peptide. The resulting recombinant DNA was introduced into the recipient strain of *Agrobacterium* according to Example 1-1. Thus, *Agrobacterium* was prepared for transferring the DNA encoding human erythropoietin as a foreign DNA.

### Example 4-2

### Plant transformation

Similarly as in Example 1-2, a strawberry was transformed by using the above-obtained *Agrobacterium* for the foreign DNA transfer according to the methods described in "Methods in Enzymology," Vol.153 (edited by Ray Wu et al., published by Academic Press Inc., 1987), chapter 16 for transforming tobacco leaves, and juvenile plant bodies was regenerated. From each juvenile plant body, a part was collected and homogenized, and the homogenates were examined for the existence of the DNA encoding human erythropoietin by a usual PCR. Plant bodies which were observed to integrate the DNA were selected as those of the transgenic strawberry.

The above-selected juvenile plant bodies of the transgenic strawberry were planted in a test garden. The strawberry fruits formed were harvested, and the harvested product were homogenized and extracted with the equal volume of an appropriate extraction buffer (pH 8.0) prepared in a usual manner. The extract was centrifuged, and the supernatant was concentrated by ultrafiltration. The concentrate was partially purified by usual methods for erythropoietin purification. The partially purified preparation was examined for erythropoietin activity by a usual assay using mouse fetal liver cells for examining the proliferation of the progenitor cells of erythroblasts. The extract was estimated to contain about 13 international units of erythropoietin activity per one milliliter.

An animal experiment was conducted with three groups of B6C3F₁ mice (10 heads per group, age of six-week-old, body weight of about 20 g to about 30 g) as follows. To the mice of group 1 (test group), phenylhydrazine was administered at 10 mg/kg-body-weight/day to induce anemia, and the fruits of the transgenic strawberry, planted and harvested similarly as above and cut into pieces, were fed at two grams by fresh weight per day per head together with a usual feed. The mice of group 2 (control group 1) received phenlyhidrazine as the test group mice and a strawberry with no foreign DNA at a similar dose of the transgenic strawberry for the test group mice. The mice of group 3 (control group 2) were fed with the usual feed only, without administering phenylhydrazine and feeding any strawberry. On day 14 from the start of this experiment, the blood cells of each mouse were counted in a usual manner, and the counts were averaged in each group. The mean value of the control group 1 was about 60% of that of the control group 2, indicating that anemia was induced in the control group 1. In the test group, the blood cell counts were apparently higher than in the control group 1 and comparable to in the control group 2. The body weights of the mice of the test group and control group 2 were not significantly different. These results indicate that human erythropoietin contained in the transgenic strawberry of this Example effected in mouse bodies to exhibit the inherent activity and modulate the mice's physiological functions to adjust the blood cell counts within a normal range. Another experiment was conducted similarly as the above experiment in which a usual feed containing an isolated preparation of human erythropoietin was used in place of the transgenic strawberry to feed mice at a daily dose corresponding to the above test group with respect to the amount of erythropoietin. In this experiment, the isolated preparation of erythropoietin did not show an apparent effect as shown in the above experiment. This result indicates that in the uses through oral routes, the transgenic plants exhibit the desired effects more surely than isolated preparations of the physiologically active proteins.

From these results, the transgenic strawberry of this Example is expected to modulate the physiological functions of humans and other mammals when used as a food, and is therefore useful as a health food to prevent various diseases in the hemocyte system as well as to alleviate disordered conditions relating to hemocyte system such as anemia, resulting from other diseases.

### Example 5

### Cut vegetables and cut fruits

From each transgenic plant obtained according to Examples 1 to 4, the edible parts were isolated, and the isolated parts were immediately washed with current water and sterilized with sodium hypochlorite in a usual manner, and thereafter cut into appropriate sizes. After washed and sterilized similarly as above, the cut pieces of the plants were soaked for five minutes in the aqueous solution of 2%(w/w) trehalose containing 1%(w/w) ethyl alcohol and 0.1%(w/w) vitamin C, according to Japanese Patent Kokai No.224,565/97, and then drained. The resulting pieces of each race of the plants were divided and packed in polyethylene bags.

These products, containing in their plant tissues physiologically active proteins of mammalian origin with improved stability, are useful as health care foods for preventing mammalian diseases. In addition, the products, of which the freshness is maintained, are useful as retail goods for supermarkets, convenience stores, etc.

As described above, this invention is established on the basis of the original finding by the present inventors that physiological functions of mammals can be modulated when the mammals ingest the transgenic plants of this invention, produced from edible plants by transforming them with a DNA encoding one or more physiologically active proteins of mammalian origin, leading to preventive effects against intractable diseases. The physiologically active proteins contained in the present transgenic plants moderately exhibit their inherent activities in the bodies of mammals that ingested the plants. The present transgenic plants are therefore useful as health foods to prevent intractable diseases easily in daily lives.

This invention, which exhibits these remarkable effects, would be very significant and contributive to the art.

While there has been described what is at present considered to be the preferred embodiments of this invention, it will be understood that the various modifications may be therein, and it is intended to cover in the appended claims all such modifications as fall within the true spirits and scope of the invention.

## Claims

1. A transgenic plant produced from an edible plant by transforming the edible plant with a DNA encoding one or more proteins selected from physiologically active proteins of mammalian origin.

2. A transgenic plant according to claim 1, wherein the physiologically active proteins of mammalian origin are mammalian cytokines.

3. A transgenic plant according to claim 2, wherein the physiologically active proteins of mammalian origin are the proteins consisting of mammalian interferons, interleukins, hematopoietic factors, and growth factors.

4. A transgenic plant according to any one of claims 1, 2 or 3, wherein the origin of the physiologically active proteins is human, bovine, mouse, or rat.

5. A transgenic plant according to any preceding claim, wherein the edible plant is a plant that is edible without cooking or heating.

6. A transgenic plant according to any preceding claim, wherein the edible plant is a plant of a family selected from the group consisting of Solanaceae, Apiaceae, Asteraceae, Brassicaceae, Cucurbitaceae, Rosaceae, Vitaceae, Ericaceae, Caricaceae, Fabaceae, Juglandaceae, and Chenopodiaceae.

7. A transgenic plant according to any preceding claim, wherein the physiologically active protein(s) is contained in an edible tissue of said plant.

8. A transgenic plant according to any preceding claims in the form of a tissue(s) isolated from the whole plant body, wherein the tissue(s) is one or more tissues selected from the group consisting of leaves, stems, roots, fruits, peels, buds, and petals.

9. A transgenic plant according to any preceding claim, which is in a processed form obtainable by a process comprising one or more steps of cutting, peeling, pulverizing, squeezing, and extracting.

10. A transgenic plant according to any preceding claim, which contains trehalose in or on the body of said transgenic plant.

11. A transgenic plant according to any preceding claim, for use as a medicament.

12. Use of a transgenic plant according to any preceding claim, for the preparation of a medicament for peroral administration.

13. Use of a transgenic plant according to any of claims 1 to 10, as a foodstuff.
